# EUROPÄISCHE PATENTSCHRIFT

(11) **EP 0 862 604 B1**
(45) Veröffentlichungstag und Bekanntmachung des Hinweises auf die Patenterteilung: **21.02.2001**
(21) Anmeldenummer: 96945750.6
(22) Anmeldetag: 25.11.1996
(51) Int. Cl.: C09K 19/32

(54) **VERWENDUNG VON IN DER PLASTISCH-KOLUMNAR DISKOTISCHEN FLÜSSIGKRISTALLINEN PHASE VORLIEGENDEN ORGANISCHEN VERBINDUNGEN ZUM TRANSPORT ELEKTRISCHER LADUNGEN**
USE OF ORGANIC COMPOUNDS PRESENT IN THE LIQUID-CRYSTALLINE PHASE DISCOTIC IN A PLASTIC-COLUMNAR MANNER FOR CONVEYING ELECTRICAL CHARGES
UTILISATION DE COMPOSES ORGANIQUES PRESENTS DANS LA PHASE A CRISTAUX LIQUIDES DISCOTIQUE DE MANIERE PLASTICO-COLONNAIRE POUR TRANSPORTER DES CHARGES ELECTRIQUES

(30) Priorität: 23.11.1995 DE 19543637
(43) Veröffentlichungstag der Anmeldung: 09.09.1998
(73) Patentinhaber: SIEMENS AKTIENGESELLSCHAFT, 80333 München (DE)
(72) Erfinder: SIMMERER, Jürgen, D-91052 Erlangen (DE); ETZBACH, Karl-Heinz, D-67227 Frankenthal (DE); SIEMENSMEYER, Karl, D-67227 Frankenthal (DE); PAULUS, Wolfgang, D-55128 Mainz (DE); SCHUHMACHER, Peter, D-68163 Mannheim (DE); GLÜSEN, Birgit, D-35037 Marburg (DE); RINGSDORF, Helmut, D-55124 Mainz (DE); WENDORFF, Joachim, H., D-35043 Marburg (DE); HAARER, Dietrich, D-95448 Bayreuth (DE); KETTNER, Andreas, D-35039 Marburg (DE)
(86) Internationale Anmeldenummer: DE9602262
(87) Internationale Veröffentlichungsnummer: WO9719142

(56) Entgegenhaltungen:
- EP-A- 0 386 576
- EP-A- 0 527 376
- DE-A- 4 429 597
- NATURE, Bd. 371, Nr. 6493, 8.September 1994, Seiten 141-143, XP000571433 ADAM D ET AL: "FAST PHOTOCONDUCTION IN THE HIGHLY ORDERED COLUMNAR PHASE OF A DISCOTIC LIQUID CRYSTAL" in der Anmeldung erwähnt
- PHYSICAL REVIEW LETTERS, Bd. 70, Nr. 4, 25.Januar 1993, Seiten 457-460, XP000345782 ADAM D ET AL: "TRANSIENT PHOTOCONDUCTIVITY IN A DISCOTIC LIQUID CRYSTAL" in der Anmeldung erwähnt

## Beschreibung

Die Erfindung betrifft die Verwendung von Verbindungen, die in der plastisch-kolumnar diskotischen flüssigkristallinen Phase vorliegen, zum Transport elektrischer Ladungen.

Weiterhin betrifft die Erfindung elektronische Bauelemente, welche die Verbindungen enthalten sowie Mischungen dieser Verbindungen mit kolumnar diskotisch helical und/oder kolumnar diskotisch flüssigkristallinen Verbindungen.

Photoleitende organische Polymere sind eine interessante Materialklasse und werden in Kopierern, Laserdruckern und Offsetdruckplatten in großem Umfang technisch eingesetzt. Von besonderem Interesse als organische Photoleiter sind diskotisch flüssigkristalline Verbindungen, wie beispielsweise Hexapentyloxytriphenylene, welche Ladungsträgerbeweglichkeiten von ca. 10⁻³ cm²/Vs aufweisen (Adam et al., Physical Review Letters 70, 457-460 (1993) sowie EP-A-0 527 376). Aus EP-A-0 386 576 sind niedermolekulare diskotisch flüssigkristalline Ladungsüberlragungs (charge transfer)-komplexe bestimmter Zusammensetzung bekannt.

Von einigen diskotisch kolumnar flüssigkristallinen Verbindungen ist bekannt, daß sie neben oder anstatt einer kolumnaren Phase auch höhergeordnete Phasen ausbilden können. In der älteren deutschen Anmeldung P4429597.9 werden beispielsweise diskotisch kolumnar flüssigkristalline Verbindungen beschrieben, welche in bestimmten Temperaturbereichen eine höher geordnete helicale Phase ausbilden. Derartige flüssigkristalline Phasen zeigen hohe Ladungsträgerbeweglichkeiten. Viele dieser bekannten organischen Photoleiter lassen bezüglich ihrer chemischen Stabilität jedoch noch zu wünschen übrig.

Aufgabe der vorliegenden Erfindung war es daher, organische Verbindungen mit flüssigkristallinen Eigenschaften bereitzustellen, die im flüssigkristallinen Zustand eine hohe Photoleitfähigkeit bzw. Ladungsträgerbeweglichkeit bei guter chemischer Stabilität aufweisen und sich für die Verwendung als Ladungstransportverbindungen in Photokopierern oder in elektronischen Bauelementen eignen.

Demgemäß wurde gefunden, daß flüssigkristalline Verbindungen, die in der plastisch kolumnar diskotischen Phase vorliegen, besonders hohe Ladungsträgerbeweglichkeiten bei guter chemischer Stabilität aufweisen und daß sich daher Verbindungen, die in dieser flüssigkristallinen Phase vorliegen, zum Transport elektrischer Ladungen besonders eignen.

Unter plastisch kolumnar diskotischer Phase ist hierbei eine flüssigkristalline Phase zu verstehen, welche einen Ordnungszustand darstellt, der zwischen dem kristallinen und dem diskotisch kolumnar flüssigkristallinen Zustand liegt. Dieser Ordnungszustand kann sowohl beim Aufheizen aus dem kristallinen Zustand als auch beim Abkühlen aus dem isotrop flüssigen oder weniger geordneten flüssigkristallinen Zustand beobachtet werden. Im diskotisch kolumnar flüssigkristallinen Zustand sind die Kolumnenachsen zwar orientiert, die einzelnen Kolumnen können sich jedoch entlang ihrer Achsen bewegen. Im plastisch kolumnar diskotischen Zustand ist diese Bewegung so stark eingeschränkt, daß bei Röntgenbeugungsexperimenten sogenannte gemischte Reflexionen (hkl) auftreten, wie sie für den kristallinen Zustand charakteristisch sind. Im Gegensatz zum kristallinen Zustand sind die plastisch kolumnar diskotischen Phasen jedoch meist viskos und nicht fest, weil die Moleküle um die Kolumnenachse rotieren können. Derartige flüssigkristalline Verbindungen lassen sich daher großflächig homogen verarbeiten und eignen sich auch für Anwendungen, für die die Dimension kristalliner Halbleiter limitierend ist. Besonders bei oligomeren Verbindungen kann die plastisch kolumnar diskotische Phase auch im glasartig erstarrten Zustand beobachtet werden.

Diese plastisch kolumnar diskotisch flüssigkristallinen Phasen zeigen nun Ladungsträgerbeweglichkeiten, die meist über 10⁻² cm²/Vs liegen. Erfindungsgemäße Verwendung können alle diskotisch flüssigkristallinen Verbindungen finden, die in bestimmten Temperaturbereichen plastisch kolumnar diskotische Phasen aufweisen. So können sich die erfindungsgemäßen Verbindungen beispielsweise von folgenden Stammkörpern ableiten:

Von Triphenylen, Dibenzopyren, Benzol, Naphthalin, Anthracen, Phenanthren, Benzophenanthren, Pentahelicen, Perylen, Decacyclen, Truxen, Rufigallol, Pyren, Fluoren, Inden, Coronen oder von entsprechenden aromatischen Systemen, in welchen ein oder mehrere Ringkohlenstoffatome durch Stickstoff, Sauerstoff oder Schwefel ersetzt sind, sowie von Tricyclochinazolin, Phthalocyanin oder Porphyrin.

Bei all diesen diskotisch flüssigkristallinen Verbindungen muß das Substitutionsmuster so gewählt werden, daß das plastisch kolumnar diskotisch flüssigkristalline Phasenverhalten auftritt. Dies ist besonders häufig der Fall, wenn das aromatische System durch kurze aliphatische Reste substituiert ist wie beispielsweise Butyloxy, Butylthio, Pentyloxy oder Pentylthio. Bei vielen der Verbindungen ist die Symmetrie der Verbindung in einer Weise gestört, daß keine Symmetrieachse senkrecht zur Molekülebene vorhanden ist, jedoch zeigen auch symmetrische Verbindungen das gewünschte flüssigkristalline Phasenverhalten wie Hexabutyloxytriphenylen. Das Vorliegen der plastisch-kolumnar diskotisch flüssigkristallinen Phase kann durch einfache polarisationsmikroskopische und differentialkalorimetrische Vorversuche (Auftreten höhergeordneter Phasen zwischen diskotisch-kolumnarer und kristalliner Phase oder zwischen isotrop flüssiger und kristalliner Phase) ermittelt und gegebenenfalls durch Röntgenbeugung bestätigt werden.

Bevorzugte Verbindungen für die erfindungsgemäßen Verwendungen sind substituierte Triphenylene, da bei diesen Verbindungen das gewünschte Phasenverhalten oft beobachtet wird. Besonders bevorzugt ist die Verwendung von Verbindungen, welche die Struktur der allgemeinen Formel I 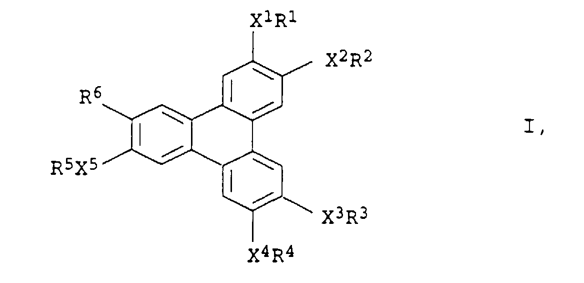 aufweisen und in der die Variablen folgende Bedeutung haben:
- X¹, X², X³, X⁴, X⁵: Sauerstoff oder Schwefel,
- R¹, R², R³, R⁴, R⁵: Butyl oder Pentyl und
- R⁶: -CN, -O-SO₂-CF₃, -OCO-tert.-Butyl, -OCO-CH₃, -O-n-C₄H₉ oder ein Substituent der allgemeinen Formel 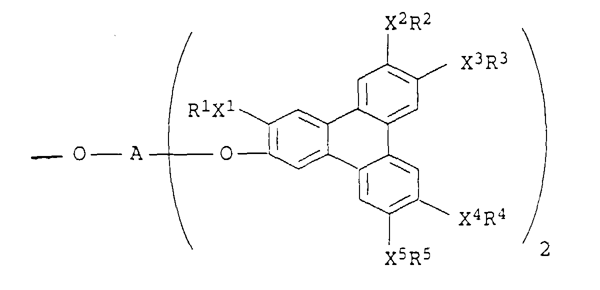
wobei A 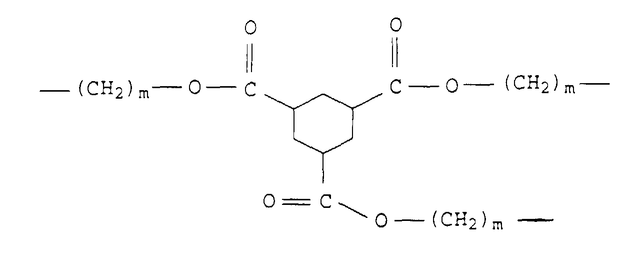 oder 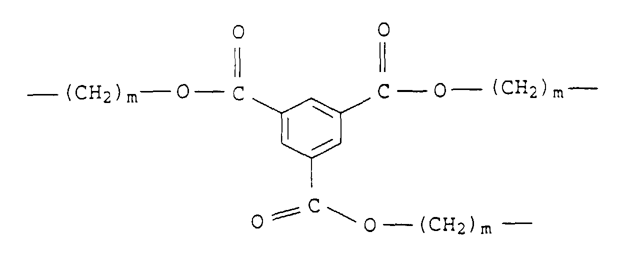 und m eine Zahl von 2 bis 6 bedeutet.

Unter diesen Verbindungen sind besonders die Penta-n-pentyloxytriphenylene, die als zusätzlichen Substituenten R⁶ eine Cyanogruppe, eine Triflatgruppe, eine Pivaloylgruppe oder eine Acetoxygruppe tragen, bevorzugt. Besonders bevorzugt ist auch das 2,3,6,7,10,11-Hexabutyloxytriphenylen, da es in einem breiten Temperaturbereich das angestrebte flüssigkristalline Verhalten zeigt und außerdem wegen seiner symmetrischen Substitution besonders leicht herstellbar ist.

Weiterhin sind unter den Verbindungen, die sich für die erfindungsgemäßen Verwendungen eignen, auch oligomere oder polymere Triphenylenderivate, z.B. solche, die über eine zentrale Cyclohexantricarbonsäuregruppe oder Benzoltricarbonsäuregruppe trimerisiert sind.

Auch doppelt oder dreifach mit einer Hydroxypropylgruppe substituierte Triphenylenderivate wie 2,6-Dihydroxypropyl-3,7,10,11-tetrapentyloxytriphenylen, das entsprechende 3,6-Isomere sowie 3,6,10-Trihydroxypropyl-2,7,11-tripentyloxytriphenylen zeigen das gewünschte flüssigkristalline Phasenverhalten.

Die erfindungsgemäßen Verbindungen finden aufgrund ihrer hohen Ladungsträgerbeweglichkeiten und ihrer geringen Dunkelleitfähigkeiten z.B. Verwendung als Photoleiter. Durch Photoionisation können in den Verbindungen freie Ladungsträger erzeugt werden, die gut transportiert werden können. Diese Eigenschaften können beispielsweise in photovoltaischen Solarzellen ausgenutzt werden, in denen die erfindungsgemäßen Verbindungen eingesetzt werden können. Weiterhin können die erfindungsgemäßen flüssigkristallinen Verbindungen in elektronischen Bauelementen wie z.B. Transistoren, Dioden oder lichtemittierenden Dioden (LED) verwendet werden.

Für die Verwendung in lichtemitierenden Dioden sind z.B. Verbindungen geeignet, welche im sichtbaren Bereich fluoreszierend sind. Weiterhin ist es möglich, die erfindungsgemäßen flüssigkristallinen Phasen mit fluoreszierenden Farbstoffen zu dotieren und so lichtemittierende Dioden herzustellen.

In elektronischen Bauelementen, in denen neben der Photoleitfähigkeit auch eine erhöhte Dunkelleitfähigkeit wünschenswert ist, lassen sich die erfindungsgemäßen flüssigkristallinen Phasen auch mit Dotierstoffen, welche die Dunkelleitfähigkeit erhöhen, versetzen. Derartige geeignete Dotierstoffe sind z.B. FeCl₃, AlCl₃, Chloranil, TNF (2,4,7-Trinitrofluorenon) oder TCNQ (2,3,5,6-Tetracyanobenzochinon)

Den der erfindungsgemäßen Verwendung zugrundeliegenden flüssigkristallinen Ordnungszustand zeigen nicht nur reine Verbindungen, sondern auch Mischungen verschiedener plastisch diskotisch kolumnar flüssigkristalliner Verbindungen sowie Mischungen dieser Verbindungen mit helical kolumnar diskotisch oder diskotisch flüssigkristallinen Verbindungen. In der Regel ist es erstrebenswert, das gewünschte flüssigkristalline Verhalten im Bereich der Raumtemperatur in einem möglichst breiten Temperaturintervall, z.B. zwischen 0 und 80°C anzutreffen. Bei reinen flüssigkristallinen Verbindungen ist dies jedoch oft nicht der Fall. Durch die Herstellung der genannten Mischungen lassen sich die Phasentemperaturen und die Phasenbreiten variieren, so daß es möglich ist, das gewünschte flüssigkristalline Verhalten in verschiedenen Temperaturbereichen zu erhalten.

Die Herstellung der erfindungsgemäß verwendeten Verbindungen ist bekannt. So lassen sich Triphenylenderivate beispielsweise nach den in der älteren deutschen Patentanmeldung 19517208.6 oder von Closs et al. in J. Chem. Soc. Perkin. Trans. I, 829 (1995), beschriebenen Methoden herstellen. Die Herstellung oligomerer Triphenylenderivate ist aus der älteren deutschen Anmeldung P 44 22 332.2 bekannt.

### Beispiele

### Charakterisierungsmethoden:

Phasenübergangstemperaturen wurden durch Differential-Scanning-Kalorimetrie mit einer Heizrate von 10 K/min ermittelt.

Die Phasenstrukturen wurden durch Weitwinkel-Röntgenstreuung (WAXS) an einem Siemens D-5000 Diffraktometer mit Nickelfilter und Cu-Kₐ-Strahlung bestimmt.

Phasenbezeichnungen:
- k: kristallin
- Dₕₒ: Diskotisch kolumnare Phase (hexagonal)
- Dₕₚ: Plastisch kolumnar diskotische Phase (Hexagonal)
- g: glasartig
- i: isotrop

### Beispiel 1

### 3,6,7,10,11-Pentapentyloxytriphenylen-2-yl-pivaloat

Zu einer Lösung von 673 mg (1 mmol) 2-Hydroxy-3,6,7,10,11-pentapentyloxytriphenylen (s. Henderson et al., Liquid Crystals 18, 191 (1995)) in 1 ml Pyridin wurden 181 mg (1,5 mmol) Pivaloylchlorid gegeben. Die Mischung wurde für 1 Stunde auf 80°C erhitzt, mit Diethylether verdünnt und mehrmals mit verdünnter HCl und Natriumcarbonatlösung extrahiert. Die Reinigung erfolgte durch Kristallisation aus Ethanol.
Ausbeute: 72 %
¹H-NMR (CDCl₃):
   δ=8,00-7,76 ppm (m, 6H, Ar-H),
   4,30-4,18 ppm (m, 10H, Ar-CH₂),
   2,00-1,82 ppm (m, 10H, ArCH₂CH₂),
   1,60-1,40 ppm (m, 20H, Ar-CH₂CH₂(CH₂)₂),
   1,46 ppm (s, 9H, C(CH₃)₃,
   1,02-0,92 (m, 15H, Ar(CH₂)₄CH₃) ;
¹³C NMR (75 MHz, CDCl₃) :
   δ= 172,0 (C=O), 149,5, 149,4, 149,1, 148,6, 139,8 (O-substituierte C-Atome des Triphenylens),
   127,7, 124,5, 123,6, 123,1, 123,0, 120,8 (innere C-Atome des Triphenylens),
   116,5, 108,1, 107,2, 106,9, 106,8, 105,5 (H-substituierte C-Atome des Triphenylens),
   69,8, 69,7, 68,5 (Ar-CH₂), 39,0 (C(CH₃)₃), 29,0 (Ar-CH₂CH₂), 28,2 (Ar-CH₂CH₂CH₂), 27,3 (C(CH₃)₃), 22,4 (Ar-(CH₂)₄CH₃).

### Phasenübergangsverhalten:

g -43°C ? -5°C Dₕₚ 65°C Dₕₒ 178°C i

### Ergebnisse der Röntgenstreuung:

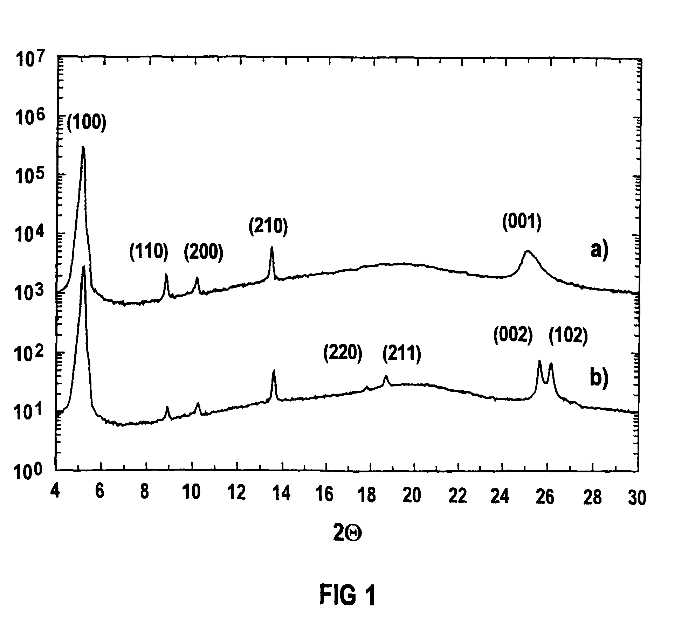

Tabelle 1 zeigt die Gitterkonstanten aₕₑₓ, (h,k,l)-Indices und d-Abstände für die Röntgenreflexionen in der Dₕₒ-Phase (88°C) und in der Dₕₚ-Phase (21°C) in Å. Das entsprechende Diffraktogramm zeigt Figur 1 (a = Dₕₒ-Phase, b = Dₕₚ-Phase). Aufgetragen ist die Intensität der Röntgenreflexion in zufälligen Einheiten gegen den Beugungswinkel.

**Tabelle 1**

| T[°C] | aₕₑₓ | (100) | (110) | (200) | (210) | | | (001) | |
|---|---|---|---|---|---|---|---|---|---|
| 88 | 20,04 | 17,36 | 10,03 | 8,73 | 6,57 | | | 3,55 | |
| | | | | | | (220) | (211) | (002) | (102) |
| 21 | 19,73 | 17,09 | 9,90 | 8,65 | 6,52 | 4,98 | 4,74 | 3,47 | 3,41 |

### Beispiel 2

### 2,3,6,7,10,11-Hexabutyloxytriphenylen

### Phasenübergangsverhalten:

k 94°C Dₕₚ 156°C i

### Röntgenstreuung:

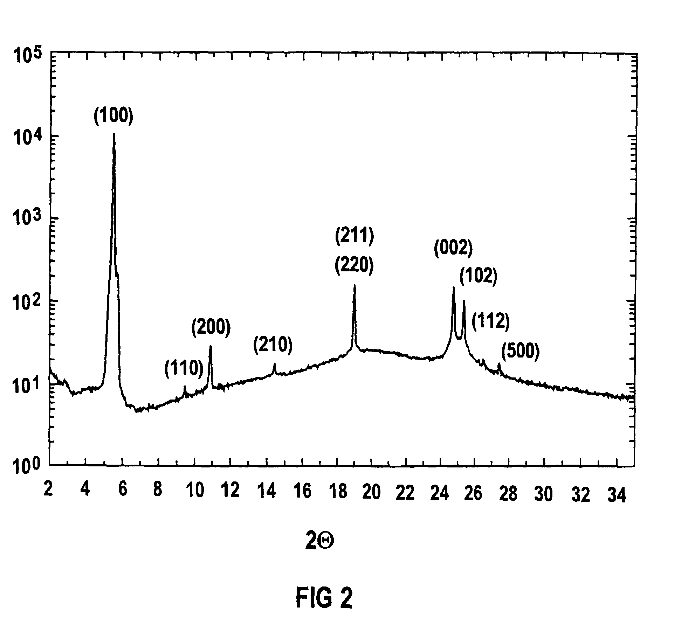

Figur 2 zeigt das Röntgendiffraktogramm von 2,3,6,7,10,11-Hexabutyloxytriphenylen bei 115°C.

### Ladungsträgerbeweglichkeit:

Die Ladungsträgerbeweglichkeit bzw. Photoleitfähigkeit wurde durch Flugzeitmessung bestimmt. Dazu wurden mit Hilfe eines Laserimpulses Ladungen in den entsprechenden Photoleiterschichten bei angelegter Grundspannung erzeugt und die Zeit bis zum Auftreten des Stromsignals gemessen. Einzelheiten der Methode sind z.B. von D.Adam etal. in Nature 371, 141-143(1994) beschrieben worden.

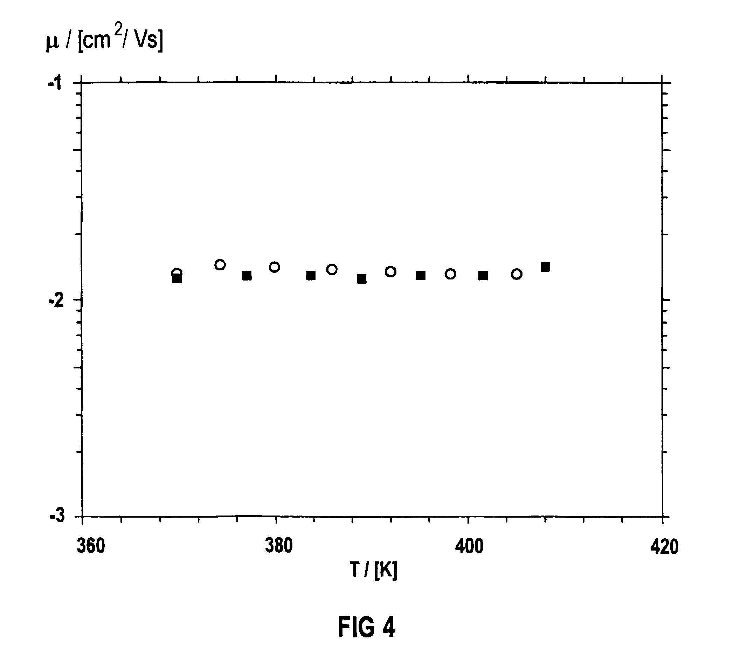

Figur 4 zeigt die Ladungsträgerbeweglichkeit µ bei verschiedenen Temperaturen. Die offenen Kreise zeigen den Verlauf bei Abkühlung, die schwarzen Quadrate den Verlauf beim Aufheizen.

### Photoleitfähigkeit:

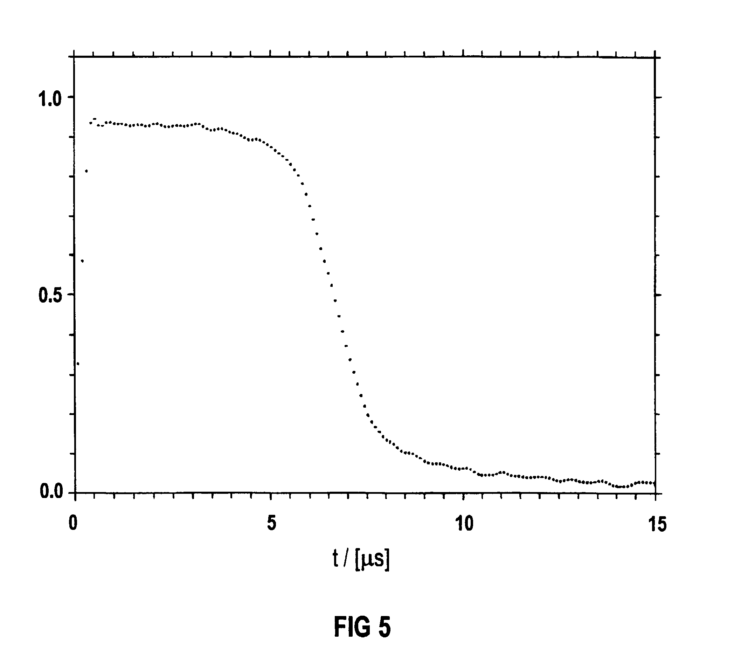

Figur 5 zeigt den zeitlichen Verlauf eines Photostromes nach einer pulsartigen Belichtung. Aufgetragen ist der Photostrom in zufälligen Einheiten gegen die Zeit.

### Beispiel 3

### 3,6,7,10,11-Pentapentyloxytriphenylen-2-yl-triflat

### Phasenübergangsverhalten:

k 54°C Dₕₚ 84°C Dₕₒ 182°C i

(Kristallisation nur aus Lösung möglich, beim Abkühlen der Reinsubstanz wurde bis -100°C keine Kristallisation und kein Glasübergang beobachtet.)

### Röntgenstreuung:

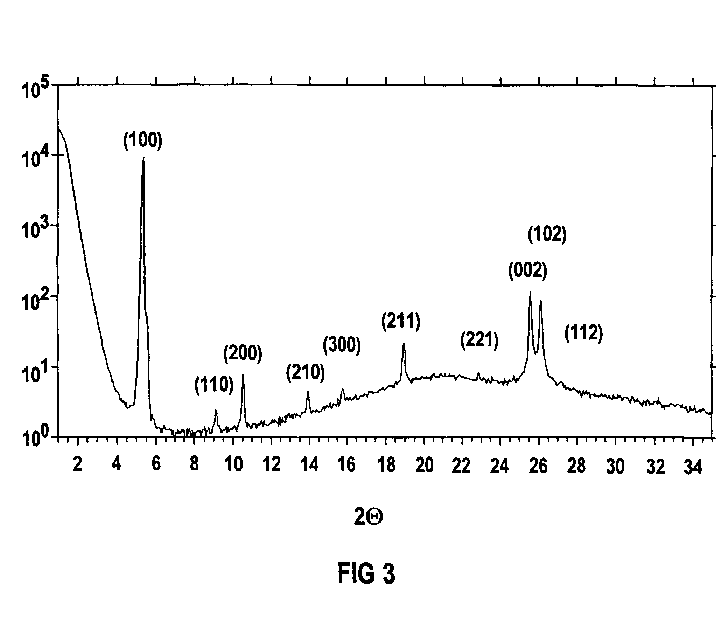

Figur 3 zeigt das Röntgendiffraktogramm der Verbindung bei 110°C.

### Beispiel 4

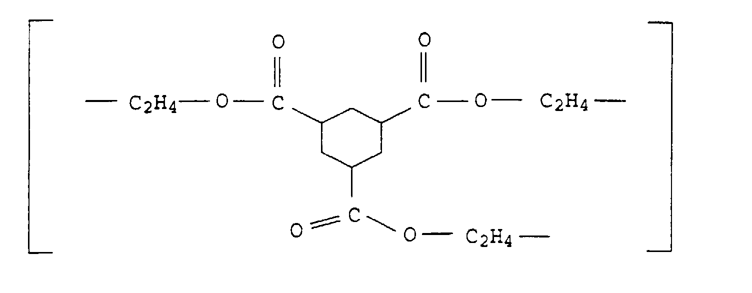

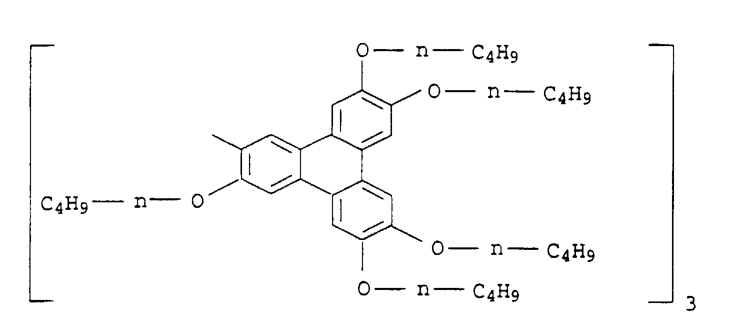

Die Herstellung der Verbindung ist nach den in der älteren deutschen Patentanmeldung P4422332.2 angegebenen Methoden möglich (die endständigen Bindungsstriche bedeuten die freien Valenzen zur Verbindung der beiden unterschiedlichen Molekülteile).

### Phasenübergangsverhalten:

k < Raumtemperatur Dhp 124°C i

### Beispiel 5

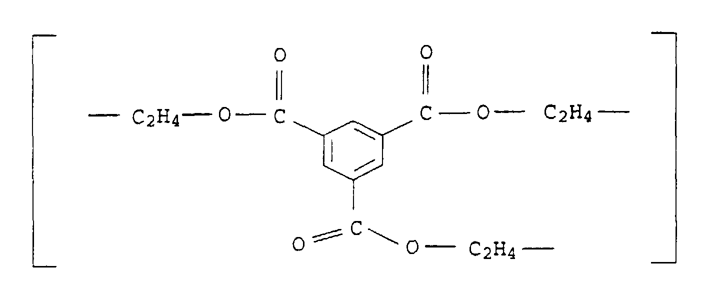

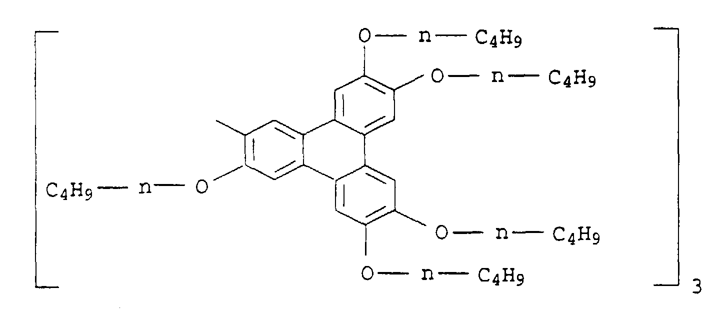

Die Herstellung der Verbindung ist nach den in der älteren deutschen Patentanmeldung P4422332.2 angegebenen Methoden möglich.

### Phasenübergangsverhalten:

k 105°C Dₕₚ 155°C i

## Patentansprüche

1. Verwendung von Verbindungen, die in der plastisch-kolumnar diskotischen flüssigkristallinen Phase vorliegen, zum Transport elektrischer Ladungen.

2. Verwendung von Verbindungen nach Anspruch 1, wobei diese Verbindungen substituierte Triphenylene sind.

3. Verwendung von Verbindungen nach Anspruch 1 und 2, wobei diese Verbindungen die Struktur der allgemeinen Formel I aufweisen 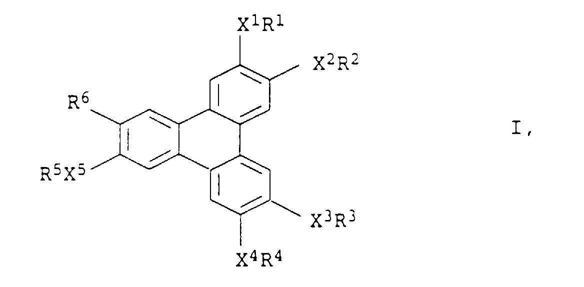 in der die Variablen folgende Bedeutung haben
X¹, X², X³, X⁴, X⁵ Sauerstoff oder Schwefel,
R¹, R², R³, R⁴, R⁵ Butyl oder Pentyl und
R⁶ -CN, -O-SO₂-CF₃, -OCO-tert.-Butyl, -OCO-CH₃, -O-n-C₄H₉ oder ein Substituent der allgemeinen Formel 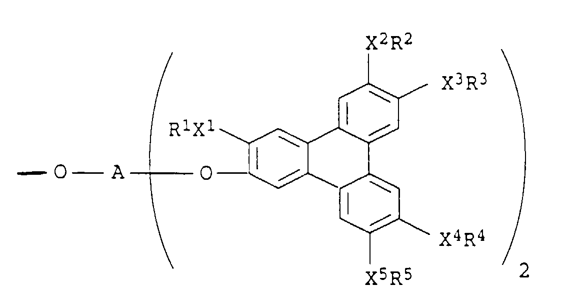
wobei A 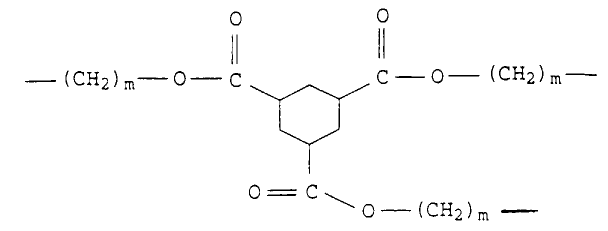 oder 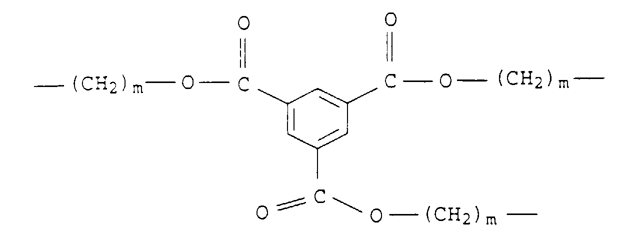 und m eine Zahl von 2 bis 6 bedeutet.

4. Verwendung von Verbindungen nach Anspruch 3, in denen
X¹, X², X³, X⁴, X⁵ Sauerstoff,
R¹, R², R³, R⁴, R⁵ n-Pentyl und
R⁶ -CN, -O-SO₂-CF₃ oder -OCO-tert.-Butyl
bedeutet.

5. Verwendung von 2,3,6,7,10,11-Hexabutyloxytriphenylen zum Transport elektrischer Ladungen nach Anspruch 1.

6. Verwendung von Verbindungen nach den Ansprüchen 1 bis 5 als Photoleiter.

7. Verwendung von Verbindungen nach den Ansprüchen 1 bis 5 in elektronischen Bauelementen.

8. Verwendung von Verbindungen nach den Ansprüchen 1 bis 5 in photovoltaischen Zellen.

9. Elektronische Bauelemente enthaltend Verbindungen gemäß den Ansprüchen 1 bis 5.

10. Mischungen von Verbindungen nach den Ansprüchen 1 bis 5 mit kolumnar diskotisch helical und/oder kolumnar diskotisch flüssigkristallinen Verbindungen.

## Claims

1. Use of compounds which are present in the plastically-columnarly discotic liquid-crystalline phase for the transfer of electric charges.

2. Use of compounds according to claim 1, wherein these compounds are substituted triphenylenes.

3. Use of compounds according to claims 1 and 2, wherein these compounds have the structure of the general formula I 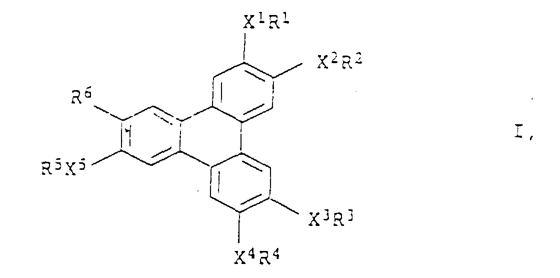 in which the variables have the following meaning:
X¹, X², X³, X⁴, X⁵ oxygen or sulphur
R¹, R², R³, R⁴, R⁵ butyl or pentyl and
R⁶ -CN, -O-SO₂-CF₃, -OCO-tert.-butyl, -OCO-CH₃, -O-n-C₄H₉ or a substituent of the general formula 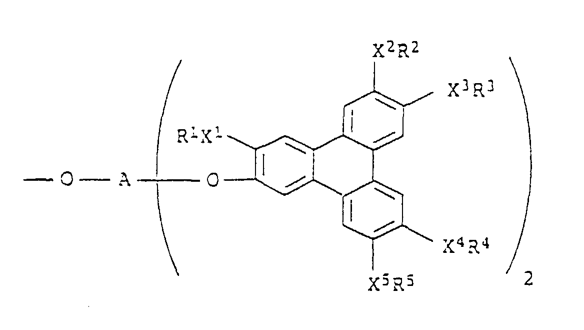
where A means 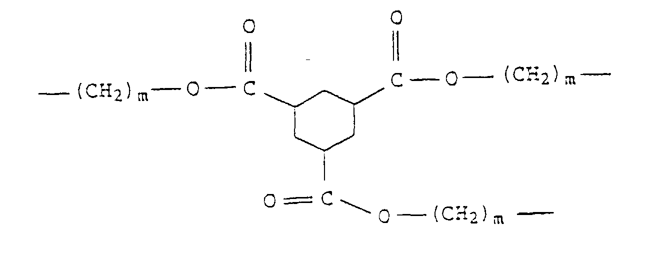 or 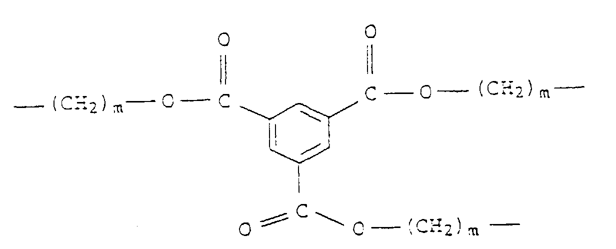 and m means a number from 2 to 6.

4. Use of compounds according to claim 3, in which
X¹, X², X³, X⁴, X⁵ means oxygen
R¹, R², R³, R⁴, R⁵ means n-pentyl and
R⁶ means -CN, -O-SO₂-CF₃ or OCO-tert.-butyl.

5. Use of 2,3,6,7,10,11-hexabutyloxytriphenylene for the transfer of electric charges according to claim 1.

6. Use of compounds according to claims 1 to 5 as photoconductors.

7. Use of compounds according to claims 1 to 5 in electronic components.

8. Use of compounds according to claims 1 to 5 in photovoltaic cells.

9. Electronic components containing compounds in accordance with claims 1 to 5.

10. Mixtures of compounds according to claims 1 to 5 with columnarly discotically helically and/or columnarly discotically liquid-crystalline compounds.

## Revendications

1. Utilisation de composés qui se trouvent en phase mésomorphe discotique plastico-colonnaire, pour transporter des charges électriques.

2. Utilisation de composés selon la revendication 1, ces composés étant des triphénylènes substitués.

3. Utilisation de composés selon la revendication 1 et 2, ces composés présentant la structure de la formule générale I 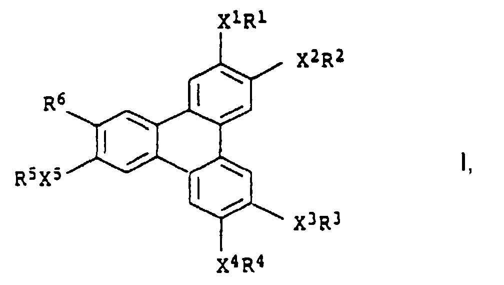 dans laquelle les variables présentent la signification suivante
X¹, X², X³, X⁴, X⁵ atome d'oxygène ou atome de soufre,
R¹, R², R³, R⁴, R⁵ groupe butyle ou groupe pentyle et
R⁶ groupe -CN, groupe -O-SO₂-CF₃, groupe -OCO-tert.-butyle, groupe -OCO-CH₃, groupe -O-n-C₄H₉ ou un substituant répondant à la formule générale 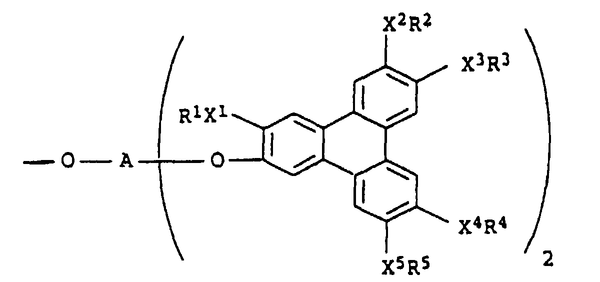
où A représente un groupe 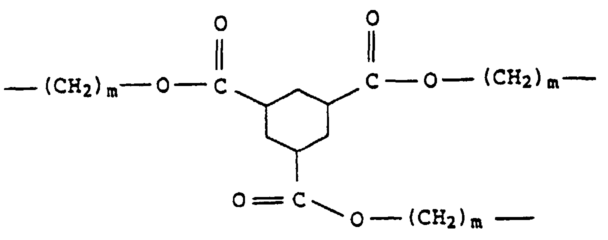 ou un groupe 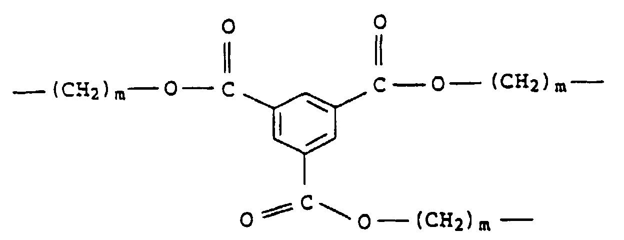 et m représente un nombre de 2 à 6.

4. Utilisation de composés selon la revendication 3, dans lesquels
X¹, X², X³, X⁴, X⁵ représentent un atome d'oxygène,
R¹, R², R³, R⁴, R⁵ représentent un groupe n-pentyle et
R⁶ représente un groupe -CN, un groupe -O-SO₂-CF₃ ou un groupe -OCO-tert.-butyle.

5. Utilisation de 2,3,6,7,10,11-hexabutyloxytriphénylène pour le transport de charges électriques selon la revendication 1.

6. Utilisation de composés selon les revendications 1 à 5, à titre de photoconducteurs.

7. Utilisation de composés selon les revendications 1 à 5 dans des composants électroniques.

8. Utilisation de composés selon les revendications 1 à 5 dans des cellules photovoltaïques.

9. Composants électroniques contenant des composés selon les revendications 1 à 5.

10. Mélanges de composés selon les revendications 1 à 5 avec des composés mésomorphes colonnaires discotiques et/ou colonnaires discotiques hélicoïdaux.
